(19) Europäisches Patentamt
European Patent Office
Office européen des brevets

(11) Publication number: 0 212 606 B1

# (12) EUROPEAN PATENT SPECIFICATION

(45) Date of publication of patent specification: 05.12.90

(51) Int. Cl.[5]: C 07 C 277/08, C 07 C 279/18

(21) Application number: 86111385.0

(22) Date of filing: 18.08.86

## (54) Spergualin-related nitrile compounds containing a phenylene group and a process for producing the same.

(30) Priority: 27.08.85 JP 186736/85

(43) Date of publication of application: 04.03.87 Bulletin 87/10

(45) Publication of the grant of the patent: 05.12.90 Bulletin 90/49

(84) Designated Contracting States: CH DE FR GB IT LI

(56) References cited:
EP-A-0 105 193
EP-A-0 153 720

CHEMICAL ABSTRACTS, vol. 104, no. 17, 28th April 1986, page 671, right-hand column, abstract no. 148642x, Columbus, Ohio, US; & JP-A-60 178 853

(73) Proprietor: MICROBIAL CHEMISTRY RESEARCH FOUNDATION
14-23, Kamiosaki 3 Chome Shinagawa-ku
Tokyo 141 (JP)

(72) Inventor: Umezawa, Hamao, Prof.
23, Toyotama-kita 4-chome Nerima-ku
Tokyo (JP)
Inventor: Takeuchi, Tomio
701A New Fuji Mansion 1-11 Higashi-gotanda 5-chome
Shingawa-ku Tokyo (JP)
Inventor: Moriguchi, Makoto
52-5, Nakakitazutsumi
Joyo-city Kyoto Prefecture (JP)
Inventor: Umeda, Yoshihisa
27-21, Jinryo 2-chome
Otsu-city Shiga Prefecture (JP)
Inventor: Nakamura, Teruya
831-6, Nomuracho
Kusatsu-city Shiga Prefecture (JP)
Inventor: Fuji, Akio
8-13, Ofuna 4-chome
Kamakura-city Kanagawa Prefecture (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 212 606 B1

Courier Press, Leamington Spa, England.

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

## Description

The present invention relates to novel Spergualin-related nitrile compounds containing a phenylene group and salts thereof which are useful as precursors for the synthesis of Spergualin-related compounds having a phenylene group, as well as to a process for producing such nitrile compounds.

Spergualin is a compound which has been isolated from the filtrate of a culture of a Spergualin-producing microorganism of the genus *Bacillus*. Spergualin has the following structure:

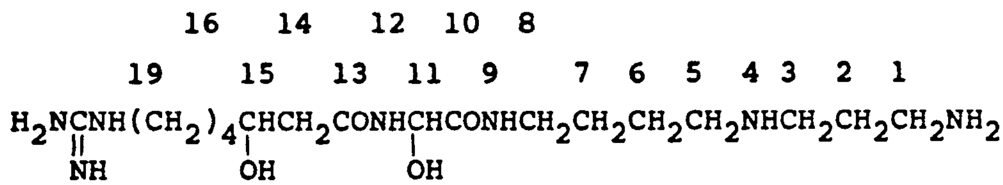

Spergualin has the effect of inhibiting the growth of both Gram-positive and Gram-negative microorganisms. It also exhibits appreciable therapeutic and life-prolonging effects in healing experiments with mouse leukemia, L—1210, mouse leukemia FL—4, Ehrlich cancer and sarcoma 180 (S—180) and has a potential for use as an anti-tumor agent (see Japanese Patent Public Disclosure No. 48957/1982). It has been reported that Spergualin can also be obtained by synthesis techniques [see The Journal of Antibiotics, *34*, 1625 (1981)].

Spergualin having a hemiacetal structure in the molecule is instable in an aqueous solution. Various studies have therefore been made with a view to obtaining Spergualin-related compounds which are stable in aqueous solutions and exhibit high activity. It was later found that Spergualin-related compounds having a phenylene group which are represented by the formula VI:

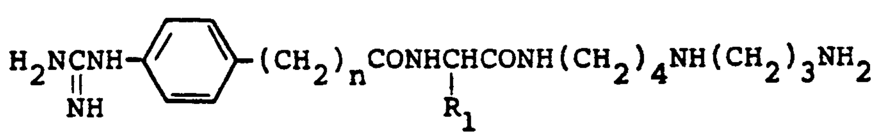

(VI)

where $R_1$ is a hydrogen atom or a hydroxylower alkyl group; and n is an integer of 3—5 are stable in aqueous solutions and exhibit high activity (see European Pat. Appl. Publ. No. 0 153 720).

Conventionally, these Spergualin-related compounds having a phenylene group are synthesized from 1,5-diprotected-1,5,10-triazadecanes of the general formula VII:

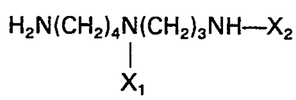

(VII)

where $X_1$ and $X_2$ which are the same or different represent an amino protecting group.

However, the compounds of the formula VII are not suitable for use in commercial operations because their synthesis requires the use of several amino protecting groups and involves several reaction steps.

Mass production of Spergualin-related compounds having a phenylene group which are represented by the formula VI could be carried out efficiently if a method for selectively acylating the primary amine at $N^{10}$ of Spermidine (1,5,10-triazadecane) present in formula VI is developed.

One object, therefore of the present invention is to provide a Spergualin-related nitrile compound having a phenylene group which is a useful precursor for the synthesis on an industrial scale of a Spergualin-related compound having a phenylene group.

Another object of the present invention is to provide a process for producing said nitrile compound.

Accordingly, the present invention relates to Spergualin-related nitrile compounds containing a phenylene group which is represented by the general formula I:

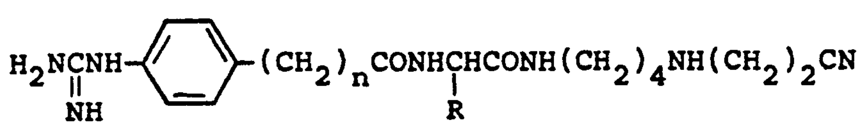

(I)

where R is a hydrogen atom, a hydroxylower alkyl group or a lower alkoxy group, and n is an integer of 3—5 and a salt thereof.

The present invention relates further to a process for producing a Spergualin-related nitrile compound

having a phenylene group which is represented by the formula I or a salt thereof, said process comprising condensing a compound of the general formula II:

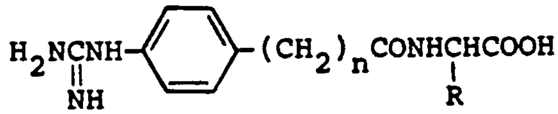 (II)

where R and n are the same as defined for the formula I, or a reactive derivative thereof, with N-2-cyanoethylbutane-1,4-diamine of the formula III:

$$H_2N(CH_2)_4NH(CH_2)_2CN \quad (III)$$

In an alternative process a compound of the formula I where R is a hydrogen atom or a hydroxylower alkyl group and n is an integer of 3—5 or a salt thereof, can be prepared by condensing a compound of the general formula IV:

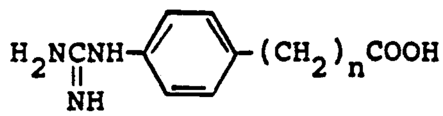 (IV)

where n is as defined above, or a reactive derivative thereof with a compound of the general formula V:

$H_2NCHCONH(CH_2)_4NH(CH_2)_2CN$ (V)
|
R

where R is a hydrogen atom or a lower hydroxyalkyl group.

The N-2-cyanoethylbutane-1,4-diamine of the general formula III is readily obtained at low cost by condensing 1,4-diamine butane and acrylonitrile [see Journal of Medicinal Chemistry, 2, 710 (1964)]. The nitrile compound of the general formula I can be synthesized from the diamine of formula III by selectively acylating the primary amine, and the present inventors have accomplished a route of synthesizing a Spergualin-related compound having a phenylene group by using said nitrile compound as a precursor.

By reducing the —CN in the nitrile compound of formula I to $—CH_2NH_2$, a compound represented by the general formula VIII:

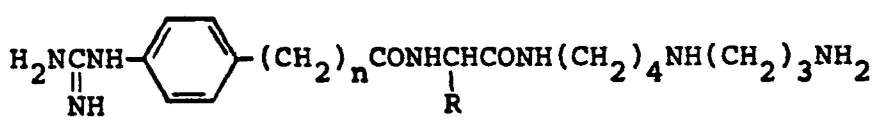 (VIII)

where R and n are the same as defined for the formula I, can be obtained. Any known reaction may be employed for reducing the —CN to $—CH_2NH_2$ without affecting the acid amide in formula I. Preferable methods include the catalytic reduction using a Raney nickel catalyst and the reduction with sodium borohydride in the presence of a transition metal salt such as cobalt chloride or nickel chloride [see Tetrahedron Letters, *52*, 4555 (1969)].

The term "hydroxyloweralkyl" as aforementioned denotes a straight or branched alkyl group having from 1 to 6, preferably 1 to 4 carbon atoms, which is substituted, preferably terminally, with a hydroxy group.

The term "lower alkoxy" as aforementioned denotes a straight or branched alkoxy group having from 1 to 6, preferably from 1 to 4 carbon atoms.

If R in the general formula I is a substituent other than a hydrogen atom, the steric configuration of the compounds may be any of the S-, R- and SR-forms. Typical examples of the compounds of the general formula I are listed in Table 1 together with their structures and names.

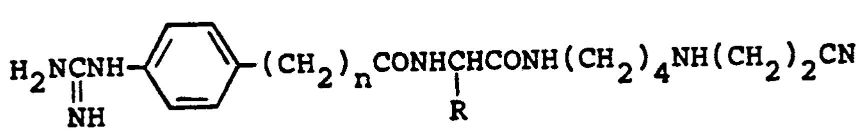 (I)

TABLE 1

| Compound No. | n | R | Name |
|---|---|---|---|
| 1 | 3 | H | 8-{N-[4-(4-guanidinophenyl)butanoyl]-glycyl}-1-cyano-3,8-diazaoctane |
| 2 | 5 | H | 8-{N-[6-(4-guanidinophenyl)hexanoyl]-glycyl}-1-cyano-3,8-diazaoctane |
| 3 | 3 | $CH_2OH$ | 8-{N-[4-(4-guanidinophenyl)butanoyl]-L-seryl}-1-cyano-3,8-diazaoctane |
| 4 | 5 | $CH_2OH$ | 8-{N-[6-(4-guanidinophenylhexanoyl]-L-seryl}-1-cyano-3,8-diazaoctane |
| 5 | 3 | $OCH_3$ | 8-{N-[4-(4-guanidinophenyl)butanoyl]-α-methoxyglycyl}-1-cyano-3,8-diazaoctane |

The compounds of the general formula I may form salts with acids. Salt-forming acids may be inorganic or organic. Although there is no particular limitation on the acids that may be used, hydrochloric acid is preferable because of the ease of its handling.

The IR absorption spectra (measured as KBa tablet), proton NMR spectra (measured in d-MeOH using TMS as a reference material) and specitif rotations of hydrochlorides of typical examples of the compound of formula I are shown in Table 2.

TABLE 2

| Compound No. | IR absorption spectra, $cm^{-1}$ | Proton NMR Spectra δ value | $[\alpha]_D^{25}$ |
|---|---|---|---|
| 1 | 3450, 2950, 2870, 2270, 1650, 1630, 1580, 1560, 1520 1460, 1420, 1260 1160, 1030, 830, 580 | 1.4 ~ 2.2 ($CH_2 \times 3$), 2.32 ($CH_2$), 2.69 ($CH_2$) 2.74 ($CH_2$), 2.9 ~ 3.4 ($NCH_2 \times 3$), 3.80 ($CH_2$), 7.20 ($CH \times 2$), 7.30 ($CH \times 2$) | — |
| 2 | 3400, 2950, 2860, 2810, 2275, 1670, 1650, 1640, 1580, 1560, 1520, 1470, 1460, 1420, 1260, 1060, 1030, 840, 670, 630 | 1.3 ~ 2.0 ($CH_2 \times 5$), 2.34 ($CH_2$), 2.68 ($CH_2$), 3.08 ($CH_2$), 2.9 ~ 3.5 ($NCH_2 \times 3$), 3.86 ($CH_2$), 7.1 ~ 7.4 ($CH \times 4$) | — |
| 3 | 3350, 2950, 2850, 2770, 2250, 1650, 1630, 1575, 1540, 1505, 1450, 1400, 1250, 1060, 820, 560, 520 | 1.4 ~ 2.2 ($CH_2 \times 3$), 2.39 ($CH_2$), 2.73 ($CH_2$), 3.00 ($CH_2$), 2.9 ~ 3.6 ($NCH_2 \times 3$) 3.79 ($CH_2$), 4.37 (CH), 7.20 ($CH \times 2$), 7.30 ($CH \times 2$) | − 13.0° (C = 1, $H_2O$) |
| 4 | 3440, 2955, 2880, 2280, 1650, 1630, 1580, 1560, 1520, 1480, 1460, 1350, 1270, 1150, 1070, 1040, 990, 960, 920, 620 | 1.2 ~ 1.9 ($CH_2 \times 5$), 2.26 ($CH_2$), 2.59 ($CH_2$), 2.9 ~ 3.4 ($NCH_2 \times 3$), 3.74 ($CH_2$), 4.30 (CH), 7.1 ~ 7.4 ($CH \times 4$), 2.97 ($CH_2$) | − 12.1° (C = 1, $H_2O$) |
| 5 | 3400, 2950, 2860, 2840, 2275, 1670, 1610, 1580, 1540, 1520, 1470, 1460, 1260, 1200, 1150, 1100, 1020, 570 | 1.5 ~ 2.2 ($CH_2 \times 3$), 2.30 ($CH_2$), 2.73 ($CH_2$), 2.9 ~ 3.5 ($NCH_2 \times 3$), 3.35 ($OCH_2$), 5.29 (CH), 7.20 ($CH \times 2$), 7.30 ($CH \times 2$) | — |

The compounds of the general formula II may be condensed with the compound of formula III by any of the routine methods employed in the formation of peptide bonds. Illustrative methods include the carbodiimide process using dicyclohexyl carbodiimide or 1-ethyl-8-(3-dimethylaminopropyl)-carbodiimide; the azide process starting from hydrazide; the mixed acid anhydride process; the active ester process using substituted or unsubstituted phenyl esters or hydroxysuccinyl imide esters; the O-acylhydroxylamine derivative process; and the N-acyl compound process. Condensation solvents may be selected from those which are commonly employed in the formation of peptide bonds. Since the compounds of the general formula II are typically used as salts with acids, amides such as dimethylformamide and dimethyl acetamide are preferably used in consideration of their ability to dissolve such salts.

When the N-2-cyanoethylbutane-1,4-diamine of the formula III is condensed with the compound of formula II, an acid, for example, hydrochloric acid may be added in an equimolar amount so that only the secondary amine in formula III is converted to a salt form, and this permits selective acylation of the primary amine. Preferential acylation of the primary amine can also be effected by using N-2-cyanoethyl-butane-1,4-diamine in an excess amount (1.2—2 mole equivalent). The condensation reaction will proceed by using equimolar amounts of the compound of the formulae II and III, but in order to attain high yields, the compound of formula III is preferably added in an excess amount.

The starting compound of the formula II is novel and may be synthesized by the following procedures. A compound of the general formula II, wherein R is a hydrogen atom or a hydroxy lower alkyl group may be synthesized by the following procedure. A compound of the general formula IV:

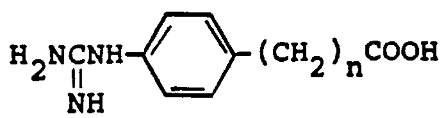

(IV)

(where n is an integer of 3—5) is condensed with a compound such as glycine or serine where a carboxyl group is protected by an appropriate protective group and, subsequently, the carboxyl-protecting group is eliminated by a routine method. The condensation reaction may be carried out by any of the aforementioned methods commonly employed in the formation of peptide bonds.

A compound of the general formula II wherein R is a lower alkoxy group may be synthesized by the following procedures: the compound of formula IV is esterified and subsequently treated with ammonia; glyoxylic acid is attached to the resulting amide so as to form a compound represented by the formula IX:

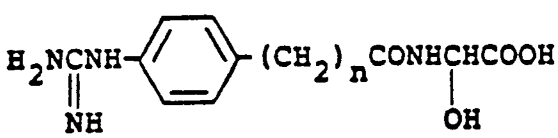

[IX]

where n is an integer of 3—5; a lower alkanol is reacted with the compound of IX in the presence of an acid catalyst so as to cause both O-alkylation and esterification; and subsequently, the ester is hydrolyzed with an alkali.

The compound of formula IV may be condensed with the compound of formula V in the third aspect of the present invention in the same manner as described in connection with the condensation reaction employed in the second aspect of the present invention. Before condensation, the compound of formula V may be treated in such a way that only the secondary amine is converted to a salt form, and this permits selective acylation of the primary amine. Preferential acylation of the primary amine can also be effected by using the compound of formula V in an excess amount (1.2—2 mole equivalent).

The starting compound of formula V is novel and may be synthesized by the following procedures: a compound such as glycine or serine wherein an amino group is protected by an appropriate protective group is condensed with the N-2-cyanoethyl-butane-1,4-diamine of formula III and, subsequently, the amino protecting group is eliminated by a routine method. Before condensation, the N-2-cyano-ethylbutane-1,4-diamine may be treated in such a way that only the secondary amine is converted to a salt form, and this permits selective acylation of the primary amine. Preferential acylation of the primary amine may also be effected by using the N-2-cyanoethylbutane-1,4-diamine in an excess amount (1.2—2 mole equivalent).

## Examples

The following examples are given for the purpose of further illustrating the present invention but are in no sense to be taken to limit the invention.

### Example 1

8-{N-[4-(4-guanidinophenyl)butanoyl]glycyl}-1-cyano-3,8-diazaoctane dihydrochloride

A mixture of 14.73 g (57.2 mmol) of 4-(4-guanidinophenyl)butyric acid hydrochloride and 7.90 g (68.6 mmol) of N-hydroxysuccinimide was dissolved in 75 ml of dimethylformamide. To the ice-cooled solution, 14.15 g (68.6 mmol) of dicyclohexyl carbodiimide in 75 ml of dimethylformamide was added dropwise. Thereafter, the mixture was stirred overnight at room temperature and the precipitating dicyclohexyl urea was filtered off. The filtrate was added dropwise to a solution of 12.81 g (64.6 mmol) of 8-(N-glycyl)-1-cyano-3,8-diazaoctane monohydrochloride in 75 ml of dimethylformamide. The resulting mixture was stirred for 1 hour at room temperature. The solvent was distilled off and the solid residue was dissolved in 1,000 ml of water. The solution was loaded onto a column packed with 300 ml of CM-Sephadex C—25 (Na form) (Pharmacia Fine Chemicals). After washing with 1,000 ml of water, the column was subjected to elution by a gradient of water (1,500 ml) and 0.5 M NaCl (1,500 ml). The active fractions were collected and vacuum-evaporated to dryness. Methanol was added to the solid residue and the insoluble NaCl was filtered off. These procedures were repeated twice. The resulting filtrate was loaded onto a column packed with 1,000 ml of Sephadex LH—20 (Pharmacia Fine Chemicals) and eluted with methanol. The active fractions were collected and concentrated under vacuum to produce 21.78 g of a syrup of 8-{N-[4-(4-guanidinophenyl)butanoyl]glycyl}-1-cyano-3,8-diazaoctane dihydrochloride in a yield of 80.2%.

Example 2

8-{N-[4-(4-guanidinophenyl)butanoyl]glycyl}-1-cyano-3,8-diazaoctane dihydrochloride

A mixture of 25.24 g (80.2 mmol) of [4-(4-guanidinophenyl)butanoyl]glycine hydrochloride and 11.1 g (96.2 mmol) of hydroxysuccinimide was dissolved in 100 ml of dimethylformamide. To the ice-cooled solution, a solution of 19.9 g (96.2 mmol) of dicyclohexyl carbodiimide in 100 ml of dimethylformamide was added dropwise. Thereafter, the mixture was stirred overnight at room temperature and the precipitating dicyclohexyl urea was filtered off. The filtrate was added dropwise to a solution of 17.0 g (120.3 mmol) of N-2-cyanoethylbutane-1,4-diamine in 80 ml of dimethylformamide, and the resulting mixture was stirred for 3 hours at room temperature. The solvent was distilled off and the solid residue was dissolved in 500 ml of 0.5 M NaCl. The resulting solution was loaded onto a column packed with 2,500 ml of Diaion HP—200 (Mitsubishi Chemical Industries Limited) adjusted with 0.5 M NaCl. The column was washed with 8,000 ml of 0.5 M NaCl, 3,000 ml of 0.25 M NaCl and 10 ml of water, and eluted with 2 × $10^4$ ml of water-methanol (9:1) and 2 × $10^4$ ml of water-methanol (8:2). The active fractions were collected and concentrated under vacuum to produce 25.74 g of a syrup of 8-{N-[4-(4-guanidinophenyl)butanoyl]glycyl}-1-cyano-3,8-diazaoctane dihydrochloride in a yield of 67.7%.

Example 3

8-{N-[6-(4-guanidinophenyl)hexanoyl]glycyl}-1-cyano-3,8-diazaoctane dihydrochloride

A mixture of 109 mg (0.38 mmol) of 6-(4-quanidinophenyl)caproic acid hydrochloride and 52.5 mg (0.46 mmol) of N-hydroxysuccinimide was dissolved in 1 ml of dimethylformamide. To the ice-cooled solution, 94.9 mg (0.46 mmol) of dicyclohexyl carbodiimide in 1 ml of dimethylformamide was added dropwise. Thereafter, the mixture was stirred for 8 hours. The stirred mixture was added dropwise to a solution of 24.7 mg (0.46 mmol) of 8-(N-glycyl)-1-cyano-3,8-diazaoctane dihydrochloride in 1 ml of dimethylformamide after it was mixed with 0.142 ml (1.01 mmol) of triethylamine. The resulting mixture was stirred overnight at room temperature. The precipitating dicyclohexyl urea was filtered off and 20 ml of water was added to the filtrate. The solution was neutralized with 1 N HCl and diluted with water to make a volume of 300 ml. The diluted solution was loaded onto a column packed with 25 ml of CM-Sephadex C—25 (Na form), washed with 250 ml of water and eluted by a gradient of 250 ml of water and 250 ml of 0.5 M NaCl. The active fractions were collected and vacuum-evaporated to dryness and the solid residue was subjected to extraction with methanol. Methanol was distilled off the extraction and the solid residue was dissolved in 0.5 M NaCl. The solution was loaded onto a column packed with 25 ml of Diaion SP 207 (Mitsubishi Chemical Industries Limited) adjusted with 0.5 M NaCl. The column was washed with 150 ml of 0.5 M NaCl and eluted with 300 ml of water and 200 ml of methanol. The active fractions were collected and concentrated under vacuum to produce 69.4 mg of a syrup of 8-{N-[6-(4-guanidinophenyl)hexanoyl]-glycyl}-1-cyano-3,8-diazaoctane dihydrochloride in a yield of 36.3%.

Example 4

8-{N-[4-(4-guanidinophenyl)butanoyl]-L-seryl}-1-cyano-3,8-diazaoctane dihydrochloride

A mixture of 107.5 g (417 mmol) of 4-(4-guanidinophenyl)butyric acid hydrochloride and 57.5 g (500 mmol) of N-hydroxysuccinimide was dissolved in 500 ml of dimethylformamide. To the ice-cooled solution, 103.2 g (500 mmol) of dicyclohexyl carbodiimide in 500 ml of dimethylformamide was added dropwise and the mixture was stirred for 5 hours at room temperature. The precipitating dicyclohexyl urea was filtered off and the filtrate was added dropwise to a solution of 150.6 g (500 mmol) of 8-N-L-seryl)-1-cyano-3,8-diazaoctane dihydrochloride in 400 ml of dimethylformamide after it was mixed with 140.5 ml (1 mol) of triethylamine. The resulting mixture was stirred for overnight at room temperature. The solvent was distilled off and the resulting solid residue was dissolved in 5,000 ml of water. The solution was loaded onto a column packed with 3,000 ml of CM-Sephadex C—25 (Na form) and, after washing with water (5,000 ml), the column was eluted with 0.2—0.3 M NaCl (1,500 ml). The active fractions were collected and vacuum-evaporated to dryness. Methanol was added to the solid residue and the insoluble NaCl was filtered off. These procedures were repeated twice. The resulting filtrate was loaded onto a column packed with 12 × $10^3$ ml of Sephadex LH—20 and eluted with methanol. The active fractions were collected and concentrated under vacuum to produce 185.4 g of a syrup of 8-{N-[4-(4-guanidinophenyl)butanoyl]-L-seryl}-1-cyano-3,8-diazaoctane dihydrochloride in a yield of 88.2%.

Example 5

8-{N-[6-(4-guanidinophenyl)hexanoyl]-L-seryl}-1-cyano-3,8-diazaoctane dihydrochloride

A mixture of 114 mg (0.40 mmol) of 6-(4-guanidinophenyl)caproic acid hydrochloride and 55.2 mg (0.48 mmol) of N-hydroxysuccinimide was dissolved in dimethylformamide (1 ml). To the ice-cooled solution, 123.8 mg (0.60 mmol) of dicyclohexyl carbodiimide in 1 ml of dimethylformamide was added dropwise and the mixture was stirred for 7 hours at room temperature. The stirred mixture was added dropwise to a solution of 182.6 mg (0.80 mmol) of 8-(N-L-seryl)-1-cyano-3,8-diazaoctane in 1 ml of dimethylformamide and the resulting mixture was stirred for 2 hours at room temperature. The precipitating dicyclohexyl urea was filtered off and the filtrate was mixed with 20 ml of water. The mixture was neutralized with 1 N HCl and diluted with 300 ml of water to make a volume of 300 ml. The dilution was loaded onto a column packed with 25 ml of CM-Sephadex C—25 (Na form) and, after being washed with

water (250 ml), the column was eluted by a gradient of water (250 ml) and 0.5 M NaCl (250 ml). The active fractions were collected and vacuum-evaporated to dryness and the solid residue was subjected to extraction with methanol. Methanol was distilled off the extract and the solid residue was dissolved in 0.5 NaCl and the solution was loaded onto a column packed with 25 ml of Diaion SP 207 adjusted with 0.5 M NaCl. After being washed with 150 ml of 0.5 M NaCl, the column was eluted with 150 ml of water and 150 ml of methanol. The active fractions were collected and concentrated under vacuum to produce 106.6 mg of a syrup of 8-{N-[6-(4-guanidinophenyl)hexanoyl]-L-seryl}-1-cyano-3,8-diazaoctane dihydrochloride in a yield of 51.5%.

Example 6

8-{N-[4-(4-guanidinophenyl)butanoyl]-α-methoxyglycyl}-1-cyano-3,8-diazaoctane dihydrochloride

A mixture of 11.03 (32.0 mmol) of 4-(4-guanidinophenyl)butanoyl-α-methoxyglycine hydrochloride and 5.50 g (48.0 mmol) of N-hydroxysuccinimide was dissolved in 50 ml of dimethylformamide. To the solution, 10.09 g (48 mmol) of dicyclohexyl carbodiimide in 50 ml of dimethylformamide was added dropwise and the mixture was stirred overnight at room temperature. The precipitating dicyclohexyl urea was distilled off and the filtrate was added dropwise to a solution of 6.80 g (48.0 mmol) of N-2-cycloethylbutane-1,4-diamine in 50 ml of dimethylformamide. The mixture was stirred overnight at room temperature. The solvent was concentrated under vacuum and the resulting solid residue was dissolved in 500 ml of water. The solution was loaded onto a column packed with 500 ml of CM-Sephadex C—25 (Na form) and, after being washed with water (2,000 ml), the column was eluted by a gradient of 1,500 ml of water and 1,500 ml of 1 M NaCl. The active fractions were collected and concentrated under vacuum. The solid residue was subjected to extraction with methanol and the solid residue was dissolved in 20 ml of water. The solution was loaded onto a column packed with 500 ml of Diaion SP 207 and, after washing with water (1,500 ml), the column was eluted with water-methanol (9:1). The active fractions were collected and concentrated under vacuum to provide 8.38 g of a syrup of 8-{N-[4-(4-guanidinophenyl)butanoyl]α-methoxyglycyl}-1-cyano-3,8-diazaoctane dihydrochloride in a yield of 52.0%.

Reference Example 1

Reduction of 8-{N-[4-(4-guanidinophenyl)butanoyl]glycyl}-1-cyano-3,8-diazaoctane dihydrochloride

21.71 g (45.8 mmol) of 8-{N-[4-(4-guanidinophenyl)butanoyl]glycyl}-1-cyano-3,8-diazaoctane dihydrochloride was dissolved in 70 ml of methanol and the solution was saturated with ammonia gas. After addition of Raney nickel (30 g), the mixture was stirred for 6 hours in a hydrogen stream (15 kg/cm$^2$(14,71 bar)) at 45°C. The catalyst was filtered off and the solvent was distilled off. The resulting solid residue was dissolved in 500 ml of water. The solution was neutralized with 2 N HCl and diluted with 1,000 ml of water. The dilution was loaded onto a column packed with 300 ml of CM-Sephadex C—25 (Na form) and, after washing with water (1,000 ml), the column was eluted with 0.2—0.3 M NaCl. The active fractions were collected and vacuum-evaporated to dryness. Methanol was added to the solid residue and the insoluble NaCl was filtered off. These procedures were repeated twice and the resulting filtrate was loaded onto a column packed with 1,000 ml of Sephadexc LH—20 and elution was conducted with methanol. The active fractions were collected and concentrated under vacuum. The resulting solid residue (13.55 g) was crystallized from methanolethanol (1:4), thereby providing 10.81 g of 10-{N-[4-(4-guanidinophenyl)-butanoyl]glycyl}-1,5,10-triazadecane trihydrochloride. Yield, 45.8%; m.p., 158—160°C.

NMR ($CH_3OH$-$d_4$): δ 1.4~2.5 ($CH_2 \times 5$), 2.66 ($CH_2$), 2.9~3.4 ($NCH_2 \times 4$), 3.78 ($CH_2$), 7.0~7.4 ($CH \times 4$)

IR (KBr): ν ($cm^{-1}$) = 3310, 2950, 1660, 1580, 1550, 1515, 1460, 1410, 1255, 1160, 1110, 1030, 830.

Reference Example 2

Reduction of 8-{N-[6-(4-guanidinophenyl)hexanoyl]glycyl}-1-cyano-3,8-diazaoctane dihydrochloride

A mixture of 69.4 mg (0.138 mmol) of 8-{N-[6-(4-guanidinophenyl)hexanoyl]glycyl}-1-cyano-3,8-diazaoctane dihydrochloride and 49.3 mg (0.207 mmol) of cobalt chloride ($CoCl_2 \cdot 6H_2O$) was dissolved in 3 ml of methanol. To the ice-cooled solution 104.4 mg (2.76 mmol) of sodium borohydride was slowly added. The mixture was stirred for 1 hour at room temperature and, after addition of water (10 ml), the solution was rendered acidic with 2N HCl to dissolve the black precipitate. Thereafter, the pH of the solution was adjusted to 7.0 with 2 N NaOH. The solvent was distilled off and the resulting solid residue was subjected to extraction with methanol. Methanol was distilled off the extract and the solid residue was dissolved in 300 ml of water. The resulting solution was loaded onto a column packed with 25 ml of CM-Sephadex C—25 (Na form) and, after washing with water (300 ml), the column was eluted by a gradient of water (250 ml) of 1 M NaCl (250 ml). The active fractions were collected and vacuum-evaporated to dryness. After addition of methanol, the insoluble NaCl was filtered off. These procedures were repeated twice and the resulting filtrate was loaded onto a column packed with 150 ml of Sephadex LH—20 and eluted with methanol. The active fractions were collected and concentrated under vacuum. The resulting solid residue (32.1 mg) was crystallized from ethanol to provide 28.4 mg of 10-{N-[6-(4-guanidinophenyl)hexanoyl]-glycyl}-1,5,10-triazadecane trihydrochloride. Yield, 37.8%; m.p., 148—151°C.

NMR ($CH_3OH$-$d_4$): δ = 1.3~2.0 ($CH_2 \times 5$), 2.0~2.4 ($CH_2 \times 2$), 2.62 ($CH_2$), 2.9~3.3 ($NCH_2 \times 4$), 3.79 ($CH_2$), 7.0~7.4 ($CH \times 4$)

IR (KBr): ν ($cm^{-1}$) = 3430, 2950, 2870, 1680, 1660, 1580, 1560, 1520, 1470, 1420, 1260.

Reference Example 3

Reduction of 8-{N-[4-(4-guanidinophenyl)butanoyl]-L-seryl}-1-cyano-3,8-diazaoctane dihydrochloride

185.4 g (368 mmol) of 8-{N-[4-(4-guanidinophenyl)butanoyl]-L-seryl}-1-cyano-3,8-diazaoctane dihydrochloride was dissolved in 400 ml of methanol and the solution was saturated with ammonia gas. After addition of Raney nickel (70 g), the saturated solution was stirred overnight in a hydrogen stream (12 kg/cm$^2$ (11,77 bar)) at 40°C. The catalyst was filtered off and the solvent was distilled off the filtrate.

The resulting solid residue was dissolved in 500 ml of water and the solution was adjusted to a pH of 7.0 with 2 N HCl. The so adjusted solution was diluted with 5,000 ml of water and loaded onto a column packed with 2.5 × 10$^3$ ml of CM-Sephadex C—25 (Na form). After washing with water 5,000 ml), the column was eluted with 0.3—0.4 M NaCl. The active fractions were collected to make a volume of 35 × 10$^3$ ml and diluted four-fold with water. The diluted solution was loaded onto a column packed with 1,500 ml of CM-Sephadex C—25 (Na form) and eluted with 0.8 M NaCl. The active fractions were collected to make a volume of 5,000 ml and vacuum-evaporated to dryness. The resulting solid residue was mixed with methanol and the insoluble NaCl was filtered off. These procedures were repeated twice and the resulting filtrate was loaded onto a column packed with 12 × 10$^3$ ml of Sephadex LH—20 and eluted with methanol. The active fractions were collected and concentrated under vacuum. The resulting solid residue was crystallized from methanol-ethanol (1:4) to provide 115.44 g of 10-{N-[4-(4-guanidinophenyl)butanoyl]-L-seryl}-1,5,10-triazadecane trihydrochloride. Yield, 57.6%; m.p., 145—146°C.

$[\alpha]_D^{25}$ −13.3° (C = 1, $H_2O$)

NMR ($CH_3OH$-$d_4$): δ = 1.4~2.5 ($CH_2$ × 5), 2.67 ($CH_2$), 2.9~3.4 ($NCH_2$ × 4), 3.75 ($CH_2$), 4.33 (CH), 7.0~7.5 (CH × 4)

IR (KBr): ν ($cm^{-1}$) = 3380, 2925, 1630, 1570, 1500, 1450, 1390, 1250, 1150, 1060.

Reference Example 4

Reduction of 8-{N-[6-(4-guanidinophenyl)hexanoyl]-L-seryl}-1-cyano-3,8-diazaoctane dihydrochloride

A mixture of 106.6 mg (0.206 mmol) of 8-{N-[6-(4-guanidinophenyl)hexanoyl]-L-seryl}-1-cyano-3,8-diazaoctane dihydrochloride and 73.5 mg (0.309 mmol) of cobalt chloride ($CoCl_2 \cdot 6H_2O$) was dissolved in methanol (3 ml). To the ice-cooled solution, 155.9 mg (4.12 mmol) of sodium borohydride was slowly added. After 1-hr stirring at room temperature, 10 ml of water was added to the mixture, which then was rendered acidic with 2 N HCl so as to dissolve the black precipitate. Thereafter, the solution was adjusted to a pH of 7.0 with 2 N NaOH and the solvent was distilled off. The resulting solid residue was subjected to extraction with methanol. Methanol was distilled off the extract and the solid residue was dissolved in 300 ml of water. The solution was loaded onto a column packed with 25 ml of CM-Sephadex C—25 (Na form) and, after washing with water (300 ml), the column was eluted by a gradient of water (300 ml) and 1 M NaCl (300 ml). The active fractions were collected and vacuum-evaporated to dryness. Methanol was added to the residue and the insoluble NaCl was filtered off. These procedures were repeated twice and the resulting filtrate was loaded onto a column packed with 150 ml of Sephadex LH—20 and eluted with methanol. The active fractions were collected and concentrated under vacuum to yield 42.8 mg of the solid residue. The residue was crystallized from ethanol to provide 40.35 mg of 10-{N-[6-(4-guanidinophenyl)-hexanoyl]-L-seryl}-1,5,10-triazedecane trihydrochloride. Yield, 35.1%; m.p., 156—160°C.

$[\alpha]_D^{25}$ −12.3° (C = 1, $H_2O$)

NMR ($CH_3OH$-$d_4$): δ~2.0 ($CH_2$ × 5), 2.0~2.5 ($CH_2$ × 2), 2.70 ($CH_2$), 2.9~3.4 ($NCH_2$ × 4), 3.78 ($CH_2$), 4.36 (CH) 7.1~7.4 (CH × 4)

IR (KBr): ν ($cm^{-1}$) = 3460, 2960, 2890, 2710, 1690, 1670, 1595, 1570, 1540, 1470, 1270, 1090.

Reference Example 5

Reduction of 8-{N-[4-(4-guanidinophenyl)butanoyl]-α-methoxyglycyl}-1-cyano-3,8-diazaoctane dihydrochloride

508 ml of 8-{N-[4-(4-guanidinophenyl)butanoyl]-α-methoxyglycyl}-1-cyano-3,8-diazaoctane dihydrochloride was saturated with ammonia gas. After addition of Raney nickel (200 mg), the solution was subjected to catalytic reduction overnight in a hydrogen stream (9 kg/cm$^2$ (8.83 bar)) at room temperature. The catalyst was filtered off and the solvent distilled off. Water (10 ml) was added to the solid residue and the pH of the solution was adjusted to 7.0 with 2 N HCl. The so adjusted solution was loaded onto a column packed with 100 ml of CM-Sephadex C—25 (Na form) and, after washing with water (100 ml), the column was eluted by a gradient of water (300 ml) and 1 M NaCl (300 ml). The active fractions were collected and concentrated under vacuum. Methanol was added to the solid residue and the insoluble NaCl was filtered off. These procedures were repeated twice and the resulting filtrate was loaded onto a column packed with 200 ml of Sephadex LH-20 and eluted with methanol. The active fractions were collected and concentrated under vacuum to provide 342 mg of 10-{N-[4-(4-guanidinophenyl)butanoyl]-α-methoxyclycyl}-1,5,10-triazadecane trihydrochloride in a yield of 62.1%.

NMR ($CH_3OH$-$d_4$): δ = 1.5~2.2 ($CH_2$ × 3), 2.30 ($CH_2$), 2.73 ($CH_2$), 2.9~3.5 ($NCH_2$ × 3, $CH_2$), 3.35 ($OCH_2$), 5.29 (CH), 7.20 (CH × 2), 7.30 (CH × 2)

IR (KBr): ν ($cm^{-1}$) = 3400, 2950, 2860, 2840, 2275, 1670, 1610, 1580, 1540, 1520, 1470, 1460, 1260, 1200, 1150, 1100, 1020, 570.

Reference Example 6

Synthesis of 8-(N-glycyl)-1-cyano-3,8-diazaoctane monohydrochloride

A mixture of 231.1 g (1.1 mol) of carbobenzoxyglycine and 139.3 g (1.21 mol) of N-hydroxysuccinimide was dissolved in 700 ml of dioxane. To the ice-cooled solution, 249.7 g (1.21 mol) of dicyclohexyl carbodiimide in 400 ml of dioxane was added dropwise. Thereafter, the mixture was stirred overnight at room temperature. The precipitating dicyclohexyl urea was filtered off and the solvent was distilled off the filtrate. The solid residue was dissolved in 500 ml of ethyl acetate. The solution was added dropwise to 233.0 g (1.65 mol) of N-2-cyanoethylbutane-1,4-diamine in 1,000 ml of ethyl acetate and the mixture was stirred overnight at room temperature. To the reaction solution, 2,000 ml of a saturated aqueous solution of $NaHCO_3$ was added and the mixture was subjected to three extractions with ethyl acetate (1,000 ml). The ethyl acetate layers were combined, washed with saturated aqueous sodium chloride twice and dried over anhydrous $Na_2SO_4$. The solvent was distilled off and the resulting solid residue (266.6 g) was dissolved in 500 ml of ethanol. Concentrated HCl was added to the ethanol solution until it became acidic. The precipitating crystal was filtered off to provide 195.9 g of 8-(carbobenzoxyglycyl)-1-cyano-3,8-diazaoctane hydrochloride. Yield, 48.3% m.p., 151—153°C. A portion (22.8 g, 68.6 mmol) of the so obtained 8-(carbobenzoxyglycyl)-1-cyano-3,8-diazaoctane hydrochloride was dissolved in 275 ml of methanol. After addition of 10% Pd-C (1.5 g), the solution was stirred in a hydrogen stream for 2 days. The catalyst was filtered off and the filtrate was concentrated under vacuum to provide 12.8 g of a syrup of 8-(N-glycyl)-1-cyano-3,8-diazaoctane monohydrochloride. This product can be immediately used as the starting material for a subsequent reaction.

Reference Example 7

Synthesis of 8-(N-L-seryl)-1-cyano-3,8-diazaoctane hydrochloride

A mixture of 598 g (2.5 mol) of carbobenzoxy-L-serine and 317 g (2.75 mol) of N-hydroxysuccinimide was dissolved in 2,500 ml of dioxane. To the ice-cooled solution, 576 g (2.75 mol) of dicyclohexyl carbodiimide in 2,000 ml of dioxane was added dropwise. Thereafter, the mixture was stirred overnight at room temperature and the precipitating dicyclohexyl urea was filtered off. The filtrate was added dropwise to a solution of 530 g (3.75 mol) of N-2-cyanoethylbutane-1,4-diamine in 1,000 ml of dioxane and the mixture was stirred overnight at room temperature. Water (1,500 ml) was added to the reaction mixture and the solution was concentrated under vacuum. To the resulting solid residue, 2,500 ml of a saturated aqueous solution of $NaHCO_3$ was added and the mixture was subjected to six extractions with ethyl acetate (1,500 ml). The ethyl acetate layers were combined, washed with saturated aqueous sodium chloride twice and dried over anhydrous $Na_2SO_4$. The solvent was distilled off and the resulting solid residue (ca. 700 g) was dissolved in 2,500 ml of ethanol. To the solution, ethanol saturated with HCl gas was added until the solution turned acidic. The precipitating crystal was filtered off to obtain 693 g of 8-(carbobenzoxy-L-seryl)-1-cyano-3,8-diazaoctane hydrochloride. Yield, 69.5%; m.p., 151—153°C.

$[\alpha]_D^{25}$ −4.6° (C = 1, $CH_3OH$).

Three hundred and fifty grams (0.88 mol) of the so obtained 8-(carbobenzoxy-L-seryl-1-cyano-3,8-diazaoctane hydrochloride was dissolved in 4,000 ml of methanol. After adding 8.75 g of 10% Pd-C, the solution was stirred in a hydrogen stream for 2 days. The catalyst was filtered off and the filtrate was concentrated under vacuum to obtain 233 g of a syrup of 8-(N-L-seryl)-1-cyano-3,8-diazaoctane monohydrochloride. This product can be immediately used as the starting material for a subsequent reaction.

Reference Example 8

Synthesis of [4-(4-guanidinophenyl)-butanoyl]glycine hydrochloride

A mixture of 35.0 g (136 mmol) of 4-(guanidinophenyl)butyric acid hydrochloride and 18.8 g (163 mmol) of N-hydroxysuccinimide was dissolved in 150 ml of dimethylformamide. To the ice-cooled solution, 33.6 g (163 mmol) of dicyclohexyl carbodiimide in 100 ml of dimethylformamide was added dropwise. Thereafter, the mixture was stirred for 5 hours at room temperature and the precipitating dicyclohexyl urea was filtered off. The filtrate was added dropwise to a solution of 22.8 g (163 mmol) of glycine ethyl ester hydrochloride and 24.8 ml of triethylamine in 200 ml of dimethylformamide, and the resulting mixture was stirred overnight at room temperature. The solvent was distilled off and the resulting solid residue was dissolved in 500 ml of water. The solution was loaded onto a column packed with 2,500 ml of Diaion HP—20 and, after washing with water, the column was eluted with 25% aqueous methanol. The active fractions were collected and concentrated under vacuum to obtain 38.0 g of a syrup of [4-[4-guanidinophenyl)butanoyl]glycine ethyl ester hydrochloride in a yield of 81.5%.

Thirty-eight grams (110 mmol) of the so obtained [4-(4-guanidinophenyl)butanoyl]glycine ethyl ester hydrochloride was dissolved in 400 ml of methanol. To the ice-cooled solution, 133 ml of 1 N NaOH was added and the mixture was stirred for 1 hour at room temperature. The pH of the mixture was adjusted to 7.0 and methanol was distilled off under vacuum. By recovering the precipitate on filter, 22.8 g of [4-(4-guanidinophenyl)butanoyl]-glycine was obtained. Yield, 74%; m.p., 257°C (with decomposition). A portion (22.3 g, 80 mmol) of the [4-(4-guanidinophenyl)butanoyl]glycine was dissolved in 80 ml of 1 N HCl and the solution was vacuum-evaporated to dryness, thereby providing 25.2 g of [4-(4-guanidinophenyl)butanoyl]-glycine hydrochloride.

Reference Example 9

Synthesis of N-[4-(4-guanidinophenyl)butanoyl]-α-methoxyglycine hydrochloride

51.6 g (0.2 mol) of 4-(4-guanidinophenyl)butyric acid was dissolved in 300 ml of methanol and, after addition of 2 g of Dowex 50W × 4 (H form) (Dow Chemical Company), the solution was boiled for 5 hours. Activated carbon was added to the reaction solution and the mixture was stirred for 30 minutes. Dowex 50W × 4 and activated carbon were filtered off and the filtrate was concentrated under vacuum to obtain 50.69 g (0.187 mol) of methyl 4-(4-guanidinophenyl)butyrate hydrochloride in a yield of 93.3%.

The so obtained methyl 4-(4-guanidinophenyl)butyrate hydrochloride (50.69 g) was dissolved in 500 ml of 28% aqueous ammonia and the solution was stirred overnight at room temperature. The reaction mixture was concentrated under vacuum and the solid residue was dissolved in 200 ml of water. The solution was loaded onto a column packed with 1,000 ml of CM-Sephadex C—25 (Na form) and, after washing with water (5,000 ml), the column was eluted with 0.5 M NaCl. The active fractions were concentrated under vacuum and the solid residue was dissolved in 100 ml of water. The solution was loaded onto a column packed with 1,000 ml of Diaion SP 207 and, after washing with water (1,200 ml), the column was eluted with water-methanol (9:1). The active fractions were concentrated under vacuum to obtain 41.94 g (0.154 mol) of 4-(4-guanidinophenyl)butaneamide hydrochloride in a yield of 77.2%.

A portion (25.7 g, 0.1 mol) of the so obtained 4-(4-guanidinophenyl)butaneamide was dissolved in 257 ml of dimethylformamide. To the solution, 10.1 g (0.11 mol) of glyoxylic acid and 257 ml of acetone were added, and the solution was refluxed on an oil bath (90°C) for 2.5 hours. The solvent was distilled off and the resulting solid residue was dissolved in 200 ml of water. The solution was loaded onto a column packed with 1,250 ml of CM-Sephadex C—25 (Na form) and eluted with water. The active fractions were collected and concentrated under vacuum to obtain 22.55 g (0.0766 mol) of N-[4-(4-guanidinophenyl)-butanoyl)-α-hydroxyglycine in a yield of 76.6%.

A portion (22.0 g, 0.748 mol) of the so obtained N-[4-(4-guanidinophenyl)butanoyl]-α-hydroxyglycine was dissolved in 580 ml of anhydrous methanol. To the ice-cooled solution, 10 ml of concentrated sulfuric acid was added and the mixture was stirred for 3 hours at room temperature. The reaction solution was added to 1,500 ml of water and the mixture was adjusted to a pH of 11.0 with 2 N NAOH. Two hours later, the mixture was adjusted to a pH of 6.5 and concentrated under vacuum. The resulting residue was loaded onto a column packed with 1,250 ml of CM-Sephadex C—25 (Na form) and eluted with water. The active fractions were collected and concentrated under vacuum to obtain 19.33 g (62.7 mmol) of N-[4-(4-guanidinophenyl)butanoyl)-α-methoxyglycine in a yield of 83.8%.

19.33 g (62.7 mmol) of the so obtained N-[4-(4-guanidinophenyl)butanoyl)-α-methoxyglycine was dissolved in 62.7 ml of 1 N HCl and the solution was concentrated under vacuum to obtain 19.84 g of N-[4-(4-guanidinophenyl)butanoyl)-α-methoxyglycine hydrochloride.

Advantages of the Invention

In the prior art techniques for the production of Spergualin-related compounds having a phenylene group, the steps of synthesizing a selective amino protected form of Spermidine and subsequently eliminating the protective group have been necessary. In accordance with the present invention, a Spergualin-related compound having a phenylene group can be synthesized from an inexpensive starting material, N-2-cyanoethylbutane-1,4-diamine, through a shorter route.

Therefore, the present invention offers an economical process for producing Spergualin-related compounds having a phenylene group.

## Claims

1. A process for the preparation of a compound of the formula I

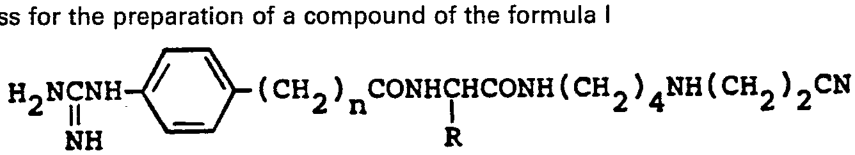

(I)

wherein R is a hydrogen atom, a hydroxy-$C_1$—$C_6$-alkyl group or a $C_1$—$C_6$-alkoxy group, and n is an integer of 3—5 and a salt thereof which comprises

a) condensing a compound of the formula II

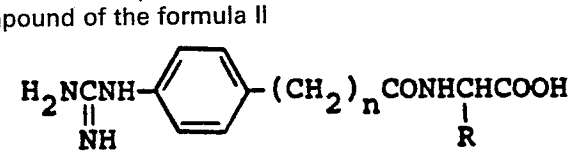

(II)

wherein R and n are as defined above, or a reactive derivative thereof, with N-2-cyanoethylbutane-1,4-diamine of the formula III:

$$H_2N(CH_2)_4NH(CH_2)_2CN \qquad (III)$$

to obtain a compound of the formula I wherein R and n are as defined above, or

b) condensing a compound of the formula IV

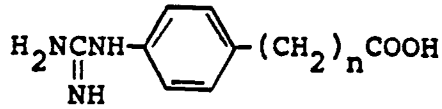

(IV)

wherein n is as defined above or a reactive derivative thereof with a compound of the general formula V

$H_2NCHCONH(CH_2)_4NH(CH_2)_2CN$
|
R

(V)

wherein R is a hydrogen atom or a hydroxy-$C_1$—$C_6$-alkyl group, to obtain a compound of the formula I wherein R and n have the aforementioned meaning.

2. A process as defined in claim 1, wherein R is hydrogen, a $C_1$—$C_4$-alkyl group terminally substituted with hydroxy, or a $C_1$—$C_4$-alkoxy group and n is the integer 3 or 5.

**Patentanspruche**

1. Verfahren zur Herstellung einer Verbindung der Formel I

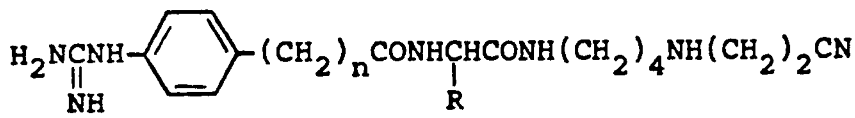

(I)

in welcher R Wasserstoff, einen Hydroxy-$C_1$—$C_6$-alkylrest oder einen $C_1$—$C_6$-Alkoxyrest bedeutet und n eine ganze Zahl von 3—5 ist und eines Salzes davon, dadurch gekennzeichnet, daß

a) eine Verbindung der formel II

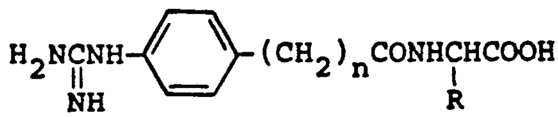

(II)

in welcher R und n wie vorher definiert sind, oder ein reaktives Derivat davon mit N-2-Cyanoäthylbutan-1,4-diamin der Formel III

$$H_2N(CH_2)_4NH(CH_2)_2CN \quad (III)$$

kondensiert wird, um eine Verbindung der Formel I, worin R und n wie vorher definiert sind, zu erhalten, oder

b) eine Verbindung der Formel IV

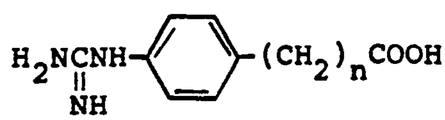

(IV)

in welcher n wie vorher definiert ist, oder ein reaktives Derivat davon, mit einer Verbindung der allgemeinen Formel V

$H_2NCHCONH(CH_2)_4NH(CH_2)_2CN$
|
R

(V)

in welcher R Wasserstoff oder einen Hydroxy-$C_1$—$C_6$-alkylrest bedeutet, kondensiert wird, um eine Verbindung der Formel I, worin R und n die vorher angegebenen Bedeutungen haben, zu erhalten.

2. Verfahren nach Anspruch 1, wobei R Wasserstoff, ein mit Hydroxy endsubstituierter $C_1$—$C_4$-Alkylrest oder ein $C_1$—$C_4$-Alkoxyrest und n eine ganze Zahl von 3 oder 5 ist.

**Revendications**

1. Procédé pour la préparation d'un composé de formule I

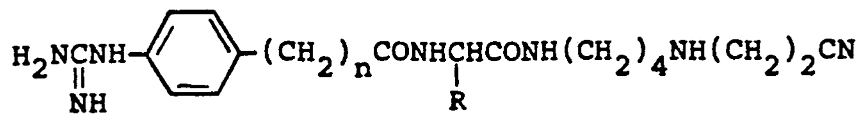 (I)

où R est un atome d'hydrogène, un groupe hydroxy(alkyl en $C_1$—$C_6$) ou un groupe alcoxy en $C_1$—$C_6$, et n est un nombre entier de 3 à 5, et d'un sel de ce composé, qui consiste à

a) condenser un composé de formule II

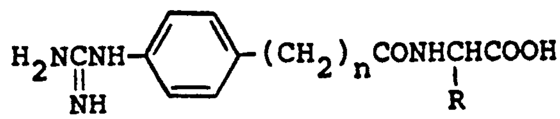 (II)

où R et n sont tels que définis ci-dessus, ou un dérivé réactif de celui-ci, avec la N-2-cyanoéthylbutane-1,4-diamine de formule III:

$$H_2N(CH_2)_4NH(CH_2)_2CN \quad \text{(III)}$$

pour obtenir un composé de formule I où R et n sont tels que définis ci-dessus, ou

b) condenser un composé de formule IV

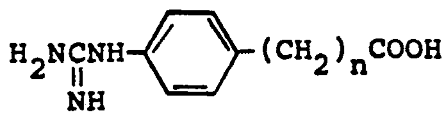 (IV)

où n est tel que défini ci-dessus, ou un dérivé réactif de celui-ci, avec un composé de formule générale V

$$\underset{\displaystyle R}{H_2N\underset{|}{C}HCONH(CH_2)_4NH(CH_2)_2CN} \quad \text{(V)}$$

où R est un atome d'hydrogène ou un groupe hydroxy(alkyle en $C_1$—$C_6$), pour obtenir un composé de formule I où R et n ont la signification susmentionnée.

2. Procédé tel que défini dans la revendication 1, dans lequel R est l'hydrogène, un groupe alkyle en $C_1$—$C_4$ substitué par un groupe hydroxyle en position terminale ou un groupe alcoxy en $C_1$—$C_4$ et n est le nombre entier 3 ou 5.